# DEMANDE DE BREVET EUROPEEN

(11) **EP 3 736 569 A1**
(43) Date de publication de la demande: **11.11.2020**
(21) Numéro de dépôt: 20183086.6
(22) Date de dépôt: 06.08.2014
(51) Int. Cl.: G01N 33/02, A23J 1/00, G01N 33/00

(54) **COMPOSITIONS DE BIOMASSE DE MICROALGUES RICHES EN PROTEINES DE QUALITE SENSORIELLE OPTIMISEE**

(30) Priorité: 07.08.2013 FR 1357843
(62) Demande divisionnaire de: 14755887.8
(71) Demandeur: Corbion Biotech, Inc., South San Francisco, CA 94080 (US)
(72) Inventeur: JEROSCH, Heike, 59940 ESTAIRES (FR); DRUON, Amandine, 59000 LILLE (FR); GUILLEMANT, Marilyne, 62120 AIRE SUR LA LYS (FR); PATINIER, Samuel, 59890 QUESNOY SUR DEULE (FR)
(74) Mandataire: Cabinet Becker et Associés

(57) **Abrégé**

L'invention est relative à une méthode pour déterminer la qualité organoleptique d'une composition de biomasse de microalgues riche en protéines comprenant la détermination de la teneur en 11 composés organiques volatils, les 11 composés organiques volatils étant le pentanal, l'hexanal, le 1-octène-3-ol, le 2-pentylfurane, l'octanal, le 3,5-octadiène-2-ol (ou 3-octène-2-one), le 3,5-octadiène-2-one, le nonanal, le 2-nonénal, le (E,E)-2,4-nonadiénal et l'acide hexanoïque.

## Description

La présente invention est relative à de nouvelles compositions de biomasse de microalgues du genre *Chlorella* riche en protéines présentant un profil sensoriel optimisé, ce qui permet de les incorporer dans des formulations alimentaires sans génération d'arômes indésirables, ainsi qu'une méthode pour évaluer le profil organoleptique d'une composition de biomasse de microalgues du genre *Chlorella* riche en protéines.

### Présentation de l'état de l'art

Il est bien connu de l'homme du métier que les chlorelles sont une source potentielle de nourriture, car elles sont riches en protéines et autres nutriments essentiels.

Elles renferment notamment 45% de protéines, 20% de matières grasses, 20% de glucides, 5% de fibres et 10% de minéraux et de vitamines.

L'utilisation des biomasses de microalgues (et principalement leurs protéines) comme aliments est de plus en plus considérée pour trouver des sources alternatives à la demande croissante en protéines animales au niveau mondial (comme rapporté par la FAO).

L'Union européenne, depuis des années, souffre par ailleurs d'un déficit structurel en protéines végétales qui s'élève, ces dernières années, à plus de 20 millions de tonnes d'équivalent soja, aujourd'hui importés d'Amérique du Sud.

La production de masse de certaines microalgues riches en protéines est alors envisagée comme une possibilité de combler ce « déficit en protéines ».

Des analyses exhaustives et des études nutritionnelles ont montré que ces protéines d'algues sont équivalentes aux protéines végétales conventionnelles, voire de meilleure qualité.

Toutefois, en raison des coûts élevés de production ainsi que des difficultés techniques pour intégrer le matériel issu de microalgues dans des préparations alimentaires organoleptiquement acceptables, la diffusion large des protéines de microalgues est encore à ses balbutiements.

Des biomasses de microalgues de différentes espèces présentant un pourcentage élevé en protéines ont été rapportées (voir le tableau 1 de Becker, Biotechnology Advances (2007)25:207-210).

Un certain nombre de demandes de brevet dans l'état de l'art, telle la demande de brevet WO 2010/045368, enseignent quant à elles qu'il est possible d'ajuster les conditions de culture de manière à augmenter encore la teneur en protéines de la biomasse de microalgues.

Cependant, lorsque l'on souhaite fabriquer industriellement des poudres de biomasse de microalgues à partir de leur biomasse, d'importantes difficultés demeurent, non seulement du point de vue technologique, mais également du point de vue du profil sensoriel des compositions produites.

En effet, si des poudres d'algues, par exemple fabriquées avec des algues cultivées photosynthétiquement dans des étangs extérieurs ou par photobioréacteurs, sont disponibles dans le commerce, elles ont une couleur vert foncée (liée à la chlorophylle) et un goût fort, désagréable.

Même formulées dans des produits alimentaires ou comme compléments nutritionnels, ces poudres d'algues communiquent toujours cette couleur verte visuellement peu attrayante au produit alimentaire ou au complément nutritionnel et ont un goût désagréable de poisson ou la saveur d'algues marines.

Par ailleurs, il est connu que certaines espèces d'algues bleues produisent naturellement des molécules chimiques odorantes telles que la géosmine (trans-1,10-diméthyle-trans-9-decalol) ou le MIB (2-méthylisoborneol), générant des odeurs terreuses ou de moisi.

Quant aux chlorelles, le descripteur communément admis dans ce domaine est le goût de « thé vert », un peu semblable à d'autres poudres végétales vertes telles que l'orge vert en poudre ou le blé vert en poudre, goût attribué à sa forte teneur en chlorophylle.

Leur saveur n'est habituellement masquée que lorsqu'elles sont mélangées avec des légumes à saveur forte ou des jus d'agrumes.

Il existe donc toujours un besoin non satisfait de disposer de compositions de biomasse de microalgues du genre *Chlorella* de qualité organoleptique convenable permettant l'utilisation de celles-ci dans des produits alimentaires plus nombreux et diversifiés.

### Résumé de l'invention

La société Demanderesse a trouvé que l'on pouvait répondre à ce besoin en proposant des compositions de biomasse de microalgues riches en protéines présentant un profil sensoriel optimisé caractérisées par la valeur globale d'arômes de 4 composés organiques volatils choisis parmi 11 composés particuliers.

Ainsi, la présente invention est tout d'abord relative à une méthode pour déterminer la qualité organoleptique d'une composition de biomasse de microalgues riche en protéines comprenant la détermination de la teneur en 11 composés organiques volatils, les 11 composés organiques volatils étant le pentanal, l'hexanal, le 1-octène-3-ol, le 2-pentylfurane, l'octanal, le 3,5-octadiène-2-ol (ou 3-octène-2-one), le 3,5-octadiène-2-one, le nonanal, le 2-nonénal, (E,E)-2,4-nonadiénal et l'acide hexanoïque.

De préférence, la biomasse de microalgues comprend plus de 50 % de protéines en poids sec de biomasse et en ce que les microalgues sont du genre Chlorella.

De préférence, la teneur de chacun de ces 11 composés organiques volatils est déterminée par SPME / GC, de préférence par SPME / GC-MS.

De préférence, la teneur en chacun de ces 11 composés organiques volatils est déterminée par la surface des pics de chromatographie après SPME / GC correspondant à chacun de ces 11 composés organiques volatils.

De préférence, la teneur en chacun de ces 11 composés organiques volatils, en particulier la surface des pics de chromatographie correspondant aux 11 composés organiques volatiles, est comparée à celle de composition de biomasse de microalgues riche en protéines de référence pour laquelle ou lesquelles les qualités organoleptiques sont définies, notamment comme inacceptable ou acceptable.

La présente invention est également relative à un procédé de définition d'un profil analytique en composés organiques volatils permettant d'évaluer la qualité organoleptique des compositions de biomasse de microalgues riches en protéines, comprenant :
- la construction d'une première matrice associant des compositions de biomasse de microalgues, dont deux témoins de qualité organoleptique acceptable et inacceptable, à l'évaluation de leurs qualités organoleptiques par un panel sensoriel d'au moins 15 personnes,
- la construction d'une seconde matrice associant à ces mêmes compositions leur caractérisation par un profil d'analyse des composés organiques volatils, et
- la corrélation de la première matrice à la seconde pour produire un modèle de relation à partir duquel les compositions présentant un profil organoleptique optimisé peuvent être ainsi caractérisées par leur profil analytique en composés organiques volatils.

De préférence, la biomasse de microalgues comprend plus de 50 % de protéines en poids sec de biomasse et en ce que les microalgues sont du genre Chlorella.

De préférence, les descripteurs de l'analyse sensorielle comprennent :
- les odeurs suivantes : végétal, purée, bouillon, beurre rance, fromage, fumier, fermenté, cacahuète et peinture et
- les couleurs suivantes : jaune et vert.

La présente invention est enfin relative à la sélection de 4 des 11 composés organiques volatils présentant un faible seuil olfactif (c'est-à-dire impactant de façon majeure l'odeur globale ressentie par les membres du panel sensoriel), pour bâtir un modèle simplifié permettant de donner une valeur d'arôme globale aux compositions de biomasse de microalgues riches en protéines. Ainsi, elle est relative à une méthode pour déterminer la qualité organoleptique d'une composition de biomasse de microalgues riche en protéines comprenant la détermination de la teneur en 4 composés organiques volatils, ces 4 composés organiques étant le 3,5-octadiène-2-ol (ou 3-octène-2-one), le 1-octène-3-ol, du 3,5-octadiène-2-one, et le (E,E)-2,4-nonadiénal.

Cette valeur d'arôme globale est alors exprimée par la somme des valeurs d'arômes individuelles du 3,5-octadiène-2-ol (ou 3-octène-2-one), du 1-octène-3-ol, du 3,5-octadiène-2-one, et du (E,E)-2,4-nonadiénal.

De préférence, la biomasse de microalgues comprend plus de 50 % de protéines en poids sec de biomasse et en ce que les microalgues sont du genre Chlorella.

Les compositions de biomasse de microalgues riches en protéines conformes à l'invention présentent donc un profil sensoriel optimisé lorsque leur valeur d'arome globale est faible, de préférence comprise entre 0 et 40 % par rapport à celle d'une composition de farine de microalgues de référence organoleptique inacceptable.

La présente invention est également relative à une méthode de sélection de compositions de biomasse de microalgues riches en protéines présentant un profil organoleptique acceptable, caractérisée en ce que la qualité organoleptique est déterminée par la méthode telle que décrite ci-dessus et que la composition est sélectionnée lorsque la valeur d'arôme globale calculée par la méthode telle que décrite ci-dessus est comprise entre 0 et 40 % par rapport à celle d'une composition de biomasse de microalgues de référence organoleptique inacceptable. De préférence, la biomasse de microalgues comprend plus de 50 % de protéines en poids sec de biomasse et en ce que les microalgues sont du genre Chlorella.

De préférence, les microalgues sont du genre Chlorella, en particulier choisies dans le groupe constitué de *Chlorella vulgaris*, *Chlorella sorokiniana* et *Chlorella protothecoides,* et plus particulièrement *Chlorella protothecoides.*

De préférence, la biomasse de microalgues comprend plus de 50 % de protéines en poids sec de biomasse.

### Description détaillée de l'invention

Au sens de l'invention, une composition de biomasse de microalgues riche en protéines présente un « profil sensoriel optimisé » ou une « qualité organoleptique optimisée », lorsque son évaluation par un panel sensoriel conclut à l'absence de défauts qui altèrent la qualité organoleptique desdites formulations alimentaires contenant ces compositions de biomasse de microalgues.

Par « qualité organoleptique » est entendue la propriété d'un aliment en termes de couleur et d'odeur.

Ces défauts sont associés à la présence de molécules spécifiques odorantes et/ou aromatiques indésirables qui se caractérisent par un seuil de perception correspondant à la valeur minimale du stimulus sensoriel nécessaire à l'éveil d'une sensation.

Le « profil sensoriel optimisé » ou « qualité organoleptique optimisé » est alors traduit par un panel sensoriel par l'obtention des meilleurs scores sur une échelle d'évaluation des 2 critères sensoriels (couleur et odeurs).

Par « environ » est entendue la valeur plus ou moins 10 % de celle-ci, de préférence plus ou moins 5 % de celle-ci. Par exemple, « environ 100 » signifie entre 90 et 110, de préférence entre 95 et 105.

Par « composition de biomasse de microalgues » est entendu une composition comprenant au minimum 50, 60, 70, 80 ou 90 % en poids sec de biomasse de microalgues. Cependant, d'autres ingrédients peuvent facultativement être compris dans cette composition.

Par « riche en protéine » est entendu une teneur en protéines dans la biomasse de plus de 50 % en poids sec, de préférence de plus de 55 %, plus préférentiellement encore de plus de 60, 65 et 70 % en poids sec de biomasse.

Au sens de la présente invention, le terme « biomasse de microalgues » doit être compris dans son interprétation la plus large et comme désignant par exemple, une composition comprenant une pluralité de particules de biomasse de microalgues. La biomasse de microalgues est dérivée de cellules de microalgues qui sont entières.

Un certain nombre de documents de l'état de l'art, comme la demande de brevet internationale WO 2010/045368, décrivent des méthodes de fabrication et d'utilisation en alimentation de la biomasse de microalgues de *Chlorella* riche en protéines.

Les microalgues dont il est question dans la présente invention sont donc des microalgues du genre *Chlorella,* plus particulièrement *Chlorella protothecoides,* plus particulièrement encore des *Chlorella* privées de pigments chlorophylliens, par toute méthode connue en soi de l'homme du métier (soit par le fait que la culture est réalisée à l'obscurité, soit parce que la souche a été mutée de manière à ne plus produire ces pigments).

Notamment, les microalgues peuvent être choisies, de manière non-exhaustive, parmi les *Chlorella protothecoides, Chlorella kessleri, Chlorella minutissima, Chlorella sp., Chlorella sorokiniama, Chlorella luteoviridis, Chlorella vulgaris, Chlorella reisiglii, Chlorella ellipsoidea, Chlorella saccarophila, Parachlorella kessleri, Parachlorella beijerinkii, Prototheca stagnora* et *Prototheca moriformis*.Ainsi, dans un mode de réalisation tout particulier, la composition de biomasse de microalgues est une composition de biomasse de *Chlorella,* et en particulier de *Chlorella protothecoides.*

Le procédé fermentaire décrit dans cette demande de brevet WO 2010/045368 permet ainsi la production d'un certain nombre de compositions de biomasse de microalgues de qualité sensorielle variable.

La méthode telle que décrite dans le présent document permet donc de sélectionner les compositions de biomasse de microalgues riches en protéines présentant un profil organoleptique acceptable, notamment pour les applications alimentaires, sans pour cela devoir organiser des évaluations organoleptiques par un panel de personnes.

### 1. Définition du profil sensoriel par la détection de 11 composés organiques volatils

La société Demanderesse a découvert que le profil sensoriel d'une composition de biomasse de microalgues riche en protéines peut être défini par la nature et le seuil de détection de molécules spécifiques odorantes, en particulier de composés organiques volatils particuliers.

En effet, elle a identifié un ensemble de 11 composés organiques volatils dont la teneur dans une composition de biomasse de microalgues riche en protéines permet de déterminer la qualité organoleptique de celle-ci.

Ces 11 composés organiques volatils sont les suivants : le pentanal, l'hexanal, le 1-octène-3-ol, le 2-pentylfurane, l'octanal, le 3,5-octadiène-2-ol (ou 3-octène-2-one), le 3,5-octadiène-2-one, le nonanal, le 2-nonénal, le (E,E)-2,4-nonadiénal et l'acide hexanoïque.

Ainsi, la présente invention est relative à une méthode pour déterminer la qualité organoleptique d'une composition de biomasse de microalgues riche en protéines comprenant la détermination de la teneur en chacun de ces 11 composés organiques volatils, les 11 composés organiques volatils étant le pentanal, l'hexanal, le 1-octène-3-ol, le 2-pentylfuran, l'octanal, le 3,5-octadiène-2-ol (ou 3-octène-2-one), le 3,5-octadiène-2-one, le nonanal, le 2-nonénal, (E,E)-2,4-nonadiénal et l'acide hexanoïque.

La méthode n'exclut pas la détermination de la teneur d'autres composés organiques volatils. Cependant, les 11 composés organiques volatils sont suffisants pour déterminer la qualité organoleptique d'une composition de biomasse de microalgues riche en protéines.

De préférence, ces composés organiques volatils sont prélevés par micro extraction en phase solide (SPME) et analysés par chromatographie en phase gazeuse GC, en particulier par GC-MS (chromatographie en phase gazeuse couplée à la spectrométrie de masse).

La fraction volatile est extraite de l'échantillon de la composition de biomasse de microalgues riche en protéines en chauffant celle-ci pendant une durée suffisante en présence d'une fibre de SPME.

La fibre peut être par exemple choisie, de manière non-exhaustive, parmi le groupe consistant en carboxen et polydiméthylsiloxane (CAR/PDMS), divinylbenzène, Carboxen et polydiméthylsiloxane (DVB/CAR/PDMS), un alliage de métal et de polydiméthylsiloxane (PDMS), une fibre Carbopack-Z® (carbone noir graphitisé), polyacrylate, Carbowax® polyéthylèneglycol (PEG), et PDMS / DVB.

De préférence, on utilise une fibre DVB/CAR/PDMS (df 50/30 µm).

La société Demanderesse recommande d'utiliser ici une technique d'extraction en voie humide (suspension aqueuse) entre 40 et 70°C, de préférence entre 50 et 65°C, en particulier environ 60 °C pendant au moins 10 minutes, de préférence au moins 15 minutes et par exemple entre 15 minutes et 1 heure.

De préférence, cette étape d'extraction est faite dans un récipient scellé. Une quantité suffisante d'échantillon doit être utilisée, par exemple au moins 1 g, notamment entre 1 et 10 g et en particulier environ 2 g.

Ces 2 g sont alors mis en suspension dans 7 ml d'eau contenant 1 g de CaCl2, 200 µl de HCl et 2,32 µg d'hexanal-d12 (étalon interne), introduit dans un flacon SPME (20 ml) scellé.

Les composés organiques volatils sont ensuite désorbés, à une température compatible avec le type de fibre SPME utilisée, par exemple entre 220 et 250 °C pour la fibre utilisée dans nos essais, plus précisément à 230°C, et injectés dans le système d'analyse.

De préférence, l'analyse est faite par chromatographie en phase gazeuse GC, en particulier par GC-MS.

Plusieurs dispositifs de GC/MS sont disponibles commercialement, par exemple le spectromètre GC/Masse Clarus (PerkinElmer, USA), le chromatographe en phase gazeuse Hewlett Packard 6890 (Hewlett Packard, USA) et le chromatographe en phase gazeuse Aglient 6890N couplé détecteur sélectif de masse Aglient 5973.

Les méthodes d'ionisation qui peuvent être utilisées en GC/MS sont par exemple la spectroscopie de masse avec ionisation en mode impact électronique (EI), en mode impact chimique (CI) l'ionisation par électronébulisateur, la décharge luminescente, la désorption par champs (FD), etc.

Les volatils extraits sont ici plus précisément désorbés dans l'injecteur du système GC-MS TSQ de Thermo Scientific, puis séparés sur une colonne CPwax52 (60m * 0.25 mm, 0.25 µm) avec le gaz Hélium à 1.5 ml/min.

Le programme de température est :
- 50°C isotherme pendant 3 min, puis
- programmation à 5°C/minute jusque 230°C,
- puis isotherme pendant 20 min.

La détection est faite par spectrométrie de masse (MS) en mode impact électronique (El) et les composés sont identifiés par comparaison avec les spectres El de la bibliothèque NIST.

Ainsi, la hauteur ou la surface du pic de chromatographie correspondant au composé organique volatil est corrélée à la quantité dudit composé.

Par « surface du pic » est entendue la surface d'un ion spécifique sous la courbe dans le chromatogramme SPME-GC/MS.

De préférence, la teneur en un des 11 composés organiques volatils est déterminée par la surface du pic de l'ion spécifique du chromatogramme SPME-GC/MS correspondant à ce composé organique volatil.

La teneur en composés organiques volatils est déterminée, notamment en comparaison à celle de produit de référence.

Ainsi, globalement, une faible teneur totale en les 11 composés organiques volatils est associée à une qualité organoleptique optimisée. A contrario, une teneur totale plus élevée en les 11 composés organiques volatils est associée à une qualité organoleptique moyenne, voire mauvaise ou inacceptable.

Par exemple, la teneur totale d'une composition d'une qualité organoleptique acceptable est faible lorsqu'elle est au minimum deux fois inférieure à celle d'une composition d'une qualité organoleptique inacceptable, par exemple au minimum 2, 3, ou 4 fois inférieure, et dans un mode de réalisation le plus exigeant, au minimum 10 fois inférieure.

### 2. Définition du panel sensoriel et choix des descripteurs

La présente invention concerne une méthode pour tester les qualités organoleptiques d'une composition de biomasse de microalgues riche en protéines comprenant l'évaluation des qualités organoleptiques par un panel de testeurs. Cette évaluation peut notamment être réalisée par les méthodes détaillées ci-dessous.

La société Demanderesse propose également un procédé de définition d'un profil analytique en composés volatils permettant d'évaluer la qualité organoleptique des compositions de biomasse de microalgues, comprenant :
- la construction d'une première matrice associant des compositions de biomasse de microalgues riches en protéines dont, de préférence, deux témoins de qualité organoleptique acceptable et inacceptable à l'évaluation de leurs qualités organoleptiques par un panel sensoriel d'au moins 15 personnes,
- la construction d'une seconde matrice associant à ces mêmes compositions leur caractérisation par un profil d'analyse des composés organiques volatils, et
- la corrélation de la première matrice à la seconde pour produire un modèle de relation à partir duquel les compositions présentant un profil organoleptique optimisé peuvent être ainsi caractérisées par leur profil analytique en composés organiques volatils.

Un panel sensoriel est formé pour évaluer les propriétés sensorielles de différents lots de compositions de biomasse de microalgues, en particulier de biomasse de *Chlorella protothecoides.*

Afin d'évaluer les propriétés sensorielles des compositions de biomasse de microalgues riches en protéines, un panel d'au moins 15 personnes, par exemple de 18 personnes, a été rassemblé.

Ce « panel expert » permet la réalisation d'analyses de type QDA® (Quantitative Descriptive Analysis), appelées classiquement « profils sensoriels » (Stone, H., Sidel, J-L., Olivier, S., Woosley, A., Singleton, R ;C ; (1974), Sensory evalution by quantitative descriptive analysis, Food Technology, 28(11), 24-33).

Comme explicité par la norme NF ISO 11035 : 1995, des séances de génération de descripteurs ont été réalisées afin de décrire de façon exhaustive les propriétés olfactives des compositions de biomasse de microalgues riches en protéines.

Pour ce faire, des lots de compositions de biomasse de microalgues riches en protéines identifiés comme très hétérogènes ont été mis en solution à 3% dans de l'eau.

Chaque panéliste évalue cette solution dans un pot en verre fermé et préalablement chauffée au bain marie à 55°C et cite toutes les odeurs que lui évoque le produit.

Lors des séances de génération des descripteurs, plus de 60 termes ont été cités par les juges pour décrire l'odeur des compositions de biomasse de microalgues riches en protéines.

La liste de descripteur a d'abord était réduite qualitativement (ex : odeur « gazon » = odeur « herbe coupée ») afin d'obtenir une liste de 16 descripteurs, puis quelques séances de QDA® ont permis encore de réduire la liste (Cf : ISO 4121 :1987) à 9 descripteurs sensoriels.

De préférence, les produits de référence tels que présentés dans le tableau suivant sont associés à chaque descripteur :

| **Descripteur** | **Référence** |
|---|---|
| **végétal** | Herbe mixée à 3% |
| **Purée** | Purée en flocon à 5.6% |
| **bouillon** | 1 KUB OR de la société MAGGI pour 2L d'eau |
| **beurre rance** | Beurre rance à 2.5% |
| **fromage** | Croûte de gorgonzola à 2% |
| **fumier** | Fumier à 2% |
| **fermenté** | Tryptone (extrait de levures) à 0.75% |
| **cacahuète** | Cacahuète broyées à 1.5% |
| **peinture** | Composition de microalgues riche en protéine très oxydée à 3% |

Le descripteur « peinture » étant organoleptiquement le plus discriminant, il est recommandé par la société Demanderesse de l'utiliser comme descripteur principal afin d'établir le classement sensoriel des différents lots de compositions de biomasse de microalgues riche en protéines produits.

### Entrainement du panel

Différents exercices ont été menés afin de former le panel à l'utilisation d'échelles d'intensité sur chaque descripteur (NF ISO 08587 :1992, ISO 08586-1 :1993, ISO 08586-2 :1994).

Les performances du panel ont été finalement validées par la réalisation d'un exercice de profil 3 fois avec les mêmes lots de compositions de biomasse de microalgues riche en protéines : le panel étant considéré comme discriminant, consensuel et répétable, (méthode décrite : Pagès, J., Lê, S., Husson, F., Une approche statistiques de la performance en analyse sensorielle descriptive , Sciences des aliments, 26(2006), 446-469), l'outil peut être utilisé pour l'évaluation sensorielle des différents lots de compositions de biomasse de microalgues riches en protéines par la méthode du QDA®.

### Le profil sensoriel

Le panel analyse chaque composition de biomasse de microalgues riche en protéines l'une après l'autre sur des échelles d'intensité pour chaque descripteur.

Le questionnaire d'une séance de profil (pour 1 échantillon) est le suivant:

### Couleur

| | clair | | | | | foncé |
|---|---|---|---|---|---|---|
| **Jaune** | □ | □ | □ | □ | □ | □ |
| **Vert** | □ | □ | □ | □ | □ | □ |

### Odeurs

| | non perçu | faible | plutôt faible | moyen | plutôt fort | fort |
|---|---|---|---|---|---|---|
| | *0* | *1* | *2* | *3* | *4* | *5* |
| **Végétal** | □ | □ | □ | □ | □ | □ |
| **Purée** | □ | □ | □ | □ | □ | □ |
| **bouillon** | □ | □ | □ | □ | □ | □ |
| **beurre rance** | □ | □ | □ | □ | □ | □ |
| **fromage** | □ | □ | □ | □ | □ | □ |
| **fumier** | □ | □ | □ | □ | □ | □ |
| **fermenté** | □ | □ | □ | □ | □ | □ |
| **cacahuète** | □ | □ | □ | □ | □ | □ |
| **peinture** | □ | □ | □ | □ | □ | □ |

Des analyses de variances (ANOVA) sont réalisées pour évaluer le pouvoir discriminant des descripteurs (descripteurs dont la p-value associée au test de Fisher est inférieure à 0,20 pour l'effet Composition dans le modèle *Descripteur* ∼ *Composition* + *Panéliste).*

L'effet Composition s'interprète comme le pouvoir discriminant des descripteurs : s'il n'y a pas d'effet (Probabilité Critique > 0,20), les différents lots de compositions n'ont pas été discriminés selon ce critère.

Plus la probabilité critique est petite, plus le descripteur est discriminant.

Le descripteur peinture est ressorti comme l'un des descripteurs les plus significatifs pour caractériser l'acceptabilité d'un lot, la note obtenue pour ce descripteur servira de classement sensoriel.

Ce classement sert alors ensuite de base pour étudier le profil d'analyse des composés organiques volatils et sélectionner des molécules responsables de la mauvaise qualité organoleptique des compositions de biomasse de microalgues.

Ainsi, le profil des composés organiques volatils des compositions de biomasse de microalgues est déterminé. Il est déterminé par toute méthode connue de l'homme du métier, et de préférence par SPME / GC-MS, tel que détaillé ci-dessus.

L'analyse des composés volatils donne des chromatogrammes en GC-MS très complexes, avec un très grand nombre de pics. Par des analyses de variances et des régressions linéaires, les composés organiques volatils qui sont le mieux corrélés aux résultats obtenus pour la matrice sensorielle et à l'odeur de peinture.

Ainsi, un profil organoleptique optimisé est associé et caractérisé par un profil analytique en composés organiques volatils.

Dans un mode de réalisation préféré, les différents composés organiques sélectionnés seront considérés par leur teneur totale, en comparaison à des compositions de référence, notamment telles que définies précédemment. En particulier, la surface totale des pics de chromatographie correspondant aux composés organiques volatils sélectionnés sera considérée et comparée.

### 3. Modèle simplifié basé sur quatre composés organiques volatils impactant l'odeur globale

Dans ce mode de réalisation préféré, la société Demanderesse a trouvé que l'on peut avantageusement établir une valeur globale d'arôme pour les compositions de biomasse de microalgues riches en protéines présentant un profil sensoriel optimisé, valeur globale basée sur 4 composés organiques volatils choisis parmi les 11 composés organiques identifiés ci-dessus..

Ces composés organiques volatils sont sélectionnés sur leur critère de faible seuil olfactif. La valeur globale d'arome est alors établie selon la relation :
Valeur globale d'arôme = ∑ □des valeurs arômes individuels du 3,5-octadiène-2-ol (ou 3-octène-2-one), du 1-octène-3-ol, du 3,5-octadiène-2-one, et du (E,E)-2,4-nonadiénal.
VA total=∑ VA(3,5-octadiène-2-ol), VA(1-octène-3-ol), VA(3,5-octadiène-2-one), et VA[(E,E)-2,4-nonadiénal],
avec *VA* = *Concentration du composé x*/ *seuil olfactif du composé x)*
Comme il sera exemplifié ci-après, les compositions de biomasse de microalgues riches en protéines qui présentent une valeur globale d'arôme faible, comprise entre 0 et 40 % par rapport à celle d'une composition de farine de microalgues de référence organoleptique inacceptable, sont assurées de présenter un profil sensoriel optimisé.

L'invention sera mieux comprise à l'aide des exemples qui suivent, lesquels se veulent illustratifs et non limitatifs.

### EXEMPLES

### Exemple 1. Définition du test sensoriel

La perception de la composition de biomasse de microalgues riche en protéines est déterminée par solubilisation dans de l'eau, milieu neutre par excellence.

Un panel sensoriel a donc été formé pour évaluer, selon la méthodologie présentée ci-avant, les propriétés sensorielles de différents lots de biomasse de microalgues riches en protéines, préparées selon l'enseignement de la demande de brevet WO 2010/045368.

18 lots de biomasse de microalgues ont été testés : lot 11, lot 12, lot 14, lot 33, lot 34, lot 42, lot 43, lot 44, lot 54, lot 81, lot 82, lot 83, lot 84, lot 85, lot 92, lot 93, lot 111, lot 112.

On présente le résultat d'une telle caractérisation des lots sur le descripteur très caractéristique d'odeur de « peinture ».

### Traitement des données

Les analyses ont été réalisées à l'aide du logiciel R (en vente libre) :
R version 2.14.1 (2011-12-22)
Copyright (C) 2011 The R Foundation for Statistical Computing
ISBN 3-900051-07-0
Platform: i386-pc-mingw32/i386 (32-bit)

Le logiciel est un environnement de travail qui nécessite le chargement de modules contenant les fonctions de calcul.

Les modules utilisés pour le traitement de données de profil sont les suivants :
- Pour l'ANOVA : Package car version 2.0-12
- Pour la régression linéaire : Package stats version 2.14.1

L'ANOVA montre des résultats significativement différents d'un produit à l'autre :
Tableau Anova (tests de Type III)
Réponse: Donnée [,peinture]

| | SCEM | ddl | valeur de F | **Pr(>F)** |
|---|---|---|---|---|
| (moyenne) | 327,94 | 1 | 234,1068 | < 2,2e-16 |
| **Composition** | 684,40 | 17 | 28,7393 | **< 2,2e-16** |
| Panéliste | 118,84 | 19 | 4,4649 | 7,097e-09 |
| Résidus | 410,44 | 293 | | |

Voici les moyennes obtenues par produits :

| Compositions | moyenne | écart type | répétition |
|---|---|---|---|
| Lot 092 | 0,00 | 0,00 | 8 |
| Lot 111 | 0,00 | 0,00 | 9 |
| Lot 12 | 0,10 | 0,10 | 10 |
| Lot 112 | 0,28 | 0,12 | 36 |
| Lot 43 | 0,29 | 0,17 | 21 |
| Lot 44 | 0,33 | 0,26 | 12 |
| Lot 33 | 0,36 | 0,36 | 11 |
| Lot 11 | 0,40 | 0,40 | 10 |
| Lot 81 | 0,96 | 0,25 | 23 |
| Lot 14 | 1,00 | 0,50 | 9 |
| Lot 82 | 1,50 | 0,34 | 24 |
| Lot 34 | 1,64 | 0,38 | 22 |
| Lot 42 | 1,96 | 0,25 | 49 |
| Lot 93 | 2,67 | 0,58 | 9 |
| Lot 84 | 3,23 | 0,36 | 22 |
| Lot 54 | 4,09 | 0,41 | 11 |
| Lot 83 | 4,24 | 0,16 | 34 |
| Lot 85 | 4,60 | 0,22 | 10 |

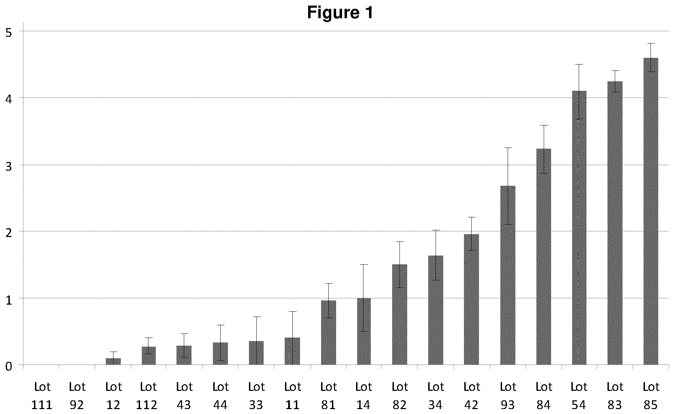

La **Figure 1** présente le classement des différents lots au regard de la note attribuée par les panélistes sur ce critère « peinture ».

Le classement est alors le suivant, par ordre de note « peinture » croissante :
Lot 92 > lot 111 > lot 12 > lot 112 > lot 43 > lot 44 > lot 33 > lot 11 > lot 81 > lot 14 > lot 82 > lot 34 > lot 42 > lot 93 > lot 84 > lot 54 > lot 83 > lot 85.
Le lot 92 est alors défini comme le Témoin de qualité organoleptique acceptable pour ce descripteur peinture.
Le lot 85 est quant à lui défini comme le Témoin de qualité organoleptique inacceptable pour ce descripteur peinture.

Cette classification organoleptique désormais établie, il est possible, efficacement selon l'invention, d'analyser le profil en SPME / GC-MS de ces échantillons afin d'identifier les cibles moléculaires de référence qui permettront de définir la qualité des compositions fabriquées.

### Exemple 2. Identification des composés organiques volatils (VOC) par SPME / GC-MS liés aux classifications organoleptiques inacceptables « odeur peinture »

Pour réaliser l'analyse en SPME / GC-MS des 18 différents lots de compositions de biomasse de microalgues, on procède comme indiqué ci-dessus en suspension aqueuse.

### Analyse des composés volatils sur produits en suspension aqueuse

Les composés volatils ont été analysés en suspension aqueuse afin de réduire l'effet matrice, et un étalon interne a été introduit.

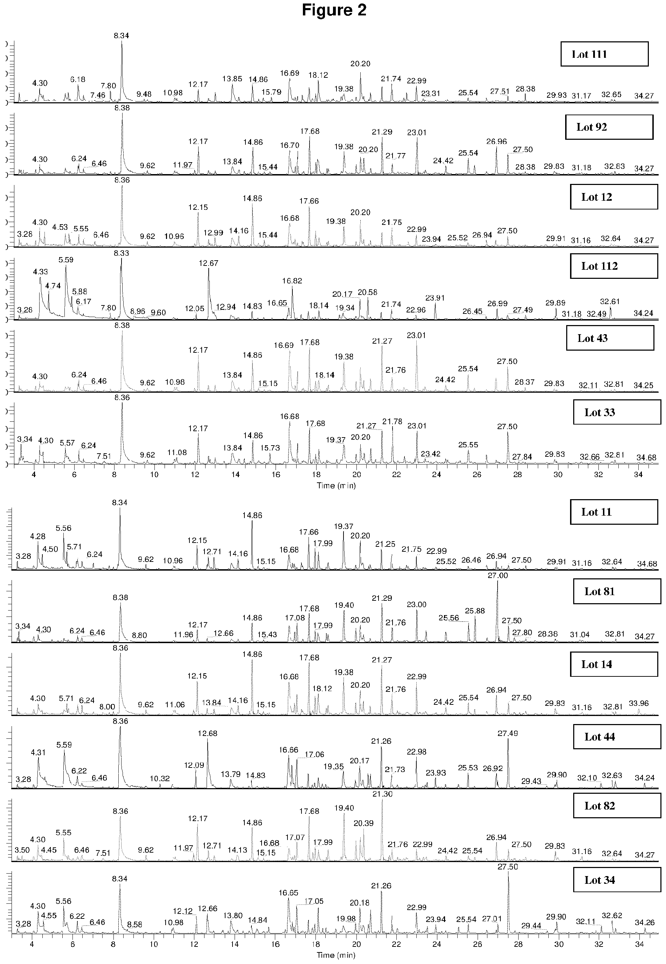

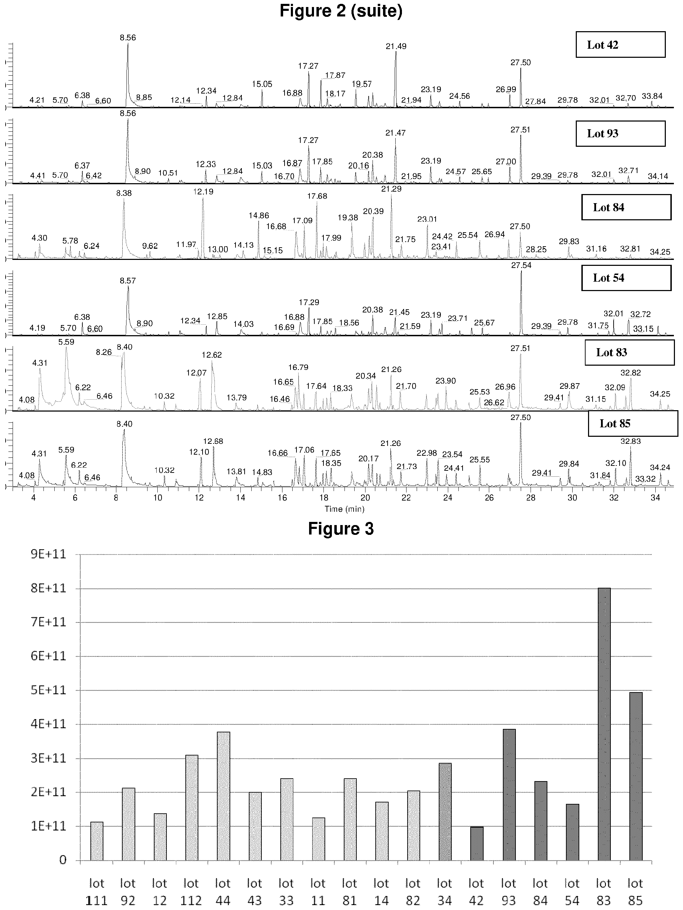

Visuellement, comme le montre la **Figure 2****,** les chromatogrammes GC-MS restent très complexes, avec un très grand nombre de composés.

La première approche consiste à comparer les profils chromatographiques, à intégrer la totalité des pics entre 3,2 à 35,0 min (TIC, « total ion current ») et vérifier si ces résultats « bruts » permettent de faire le lien avec le classement sensoriel.

La comparaison des profils chromatographiques et l'intégration de la totalité des pics entre 3,2 à 35,0 min (TIC, « total ion current ») - voir **Figure 3** - ne permettent pas de faire le lien avec le classement sensoriel.

A cause de la grande complexité des chromatogrammes, il est difficile de distinguer visuellement les produits acceptables des inacceptables.

L'intégration des surfaces des chromatogrammes ne permet pas non plus de trancher nettement entre échantillons acceptables et inacceptables.

De plus, cette approche des données brutes ne permet pas de savoir quel ou quels composé(s) volatil(s) est (sont) responsable(s) des off-notes ou goûts ou odeurs indésirables, ni de suivre spécifiquement leur apparition, ni d'avoir des renseignements sur leur voie de formation.

Une seconde approche a consisté à compléter la liste des composés organiques volatils du modèle précédent en listant les composés volatils identifiés sur les chromatogrammes SPME / GC-MS qui semblent accompagner les classifications organoleptiques.

L'analyse GC-MS-Olfactométrie de six échantillons avait fait ressortir certains composés volatils, majoritairement des aldéhydes issus de la dégradation de la fraction lipidique des compositions de biomasse de microalgues riches en protéines, qui seraient responsables des off-notes ou goûts ou odeurs indésirables.

Dans cette deuxième approche, il a alors été décidé de suivre certaines de ces molécules sélectionnées par GC-MS-Olfactométrie et par GC-MS des différents produits.

Pour sélectionner les composés organiques volatils représentatifs, on réalise une série d'analyses de la variance pour ne garder que les composés organiques volatils qui diffèrent effectivement d'une composition à l'autre compte tenue de la variabilité de la mesure SPME - GC/MS.

Le modèle est le suivant : *Composé organique volatil* ∼ *Composition* ; on ne conserve que les composés pour lesquels la probabilité critique associée au test de Fisher est inférieure à 0,05.

On présente ici 2 exemples d'ANOVA sur les composés 2-nonénal et 3.5-octadiène-2-one-2 :
2-nonénal

**Tableau Anova (tests de Type III)**

| | SCEM ddl | valeur de F | **Pr(>F)** |
|---|---|---|---|
| (moyenne) | 292,13 1 | 72,8516 | 3,51e-06 |
| **Composition** | 664,79 17 | 9,7522 | **0,0002394** |
| Résidus | 44,11 11 | | |

3,5-octadiène-2-one (pic 2)

**Tableau Anova (tests de Type III)**

| | SCEM ddl | valeur de F | **Pr(>F)** |
|---|---|---|---|
| (moyenne) | 56344 1 | 20,0633 | 0,0009326 |
| **Composition** | 72570 17 | 1,5201 | **0,2424099** |
| Résidus | 30891 11 | | |

Il apparait sur le premier composé organique volatil (le 2-nonénal) que l'effet composition est significatif (probabilité critique <0,00025), ce qui signifie qu'il y a une différence significative entre les produits compte tenu de la variabilité de la mesure.

Sur le 2ème composé (3,5-octadiène-2-one, pic 2), l'effet composition n'est pas significatif (probabilité critique > 0,05). On gardera donc pour l'étude le 2-nonénal mais pas le 3,5-octadiène-2-one.

Après cette première sélection de composés volatils, des modèles de régressions linéaires sont établis : il s'agit d'expliquer la variable « peinture » par chaque composé un à un.

On construit donc autant de modèle qu'il y a de composés. Le modèle est le suivant : *Peinture* ∼ *Composé.*

Afin de sélectionner la liste finale de composés identifiés comme responsables des classifications organoleptiques inacceptables (off-notes) observées, on ne gardera que les composés pour lesquels la probabilité critique associée au test de Student est inférieure à 0,05 (test de nullité du coefficient de régression linéaire).

Le R² associé au modèle est un indicateur pour quantifier le pourcentage de variabilité expliqué par le composé. Il peut ne pas être très élevé mais être significatif, c'est pourquoi on choisit de sélectionner les composés en fonction de la probabilité critique (afin de ne pas négliger un composé qui influe peu mais significativement sur l'odeur peinture décrite par le panel).

Coefficients:

| | *Estimateur* | *Std valeur* | valeur de t | **Pr(>\|t\|)** |
|---|---|---|---|---|
| *(ordonnée à l'origine)* | -0,669037 | 0,138335 | -4,836 | 0,000182 |
| **Hexanal** | 0,019196 | 0,002593 | 7,404 | **1,49e-06** |
| Erreur standard résiduelle: 0,4444 on 16 degrés de liberté | | | | |
| R² Multiple: 0,7741, | R² ajusté: 0,76 | | | |
| *Statistique* F: 54,82 sur 1 et 16 degrés de liberté, probabilité critique: 1,491e-06 | | | | |

Coefficients:

| | *Estimateur* | *Std valeur* | valeur de t | **Pr(>\|t\|)** |
|---|---|---|---|---|
| *(ordonnée à l'origine)* | -0,45338 | 0,24852 | -1,824 | 0,0868 |
| **3,5-octadiène-2-one (pic 1)** | 0,04346 | 0,01614 | 2,693 | **0,0160** |
| Erreur standard résiduelle: 0,7756 on 16 degrés de liberté | | | | |
| R² Multiple: 0,3119, | R² ajusté: 0,2689 | | | |
| *Statistique* F: 7,252 sur 1 et 16 degrés de liberté, probabilité critique: 0,016 | | | | |

Pour ces 2 composés, l'hexanal et le 3,5-octadiène-2-one (pic 1), la probabilité critique est inférieure à 0,05, donc ils sont corrélés à l'odeur de peinture décrite par le panel.

Les 11 composés de l'étude se trouvent être bien corrélés au descripteur « peinture ».

Ces 11 molécules sélectionnées sont listées dans le tableau ci-dessous :

| | Molécule | Temps rétention (min) | Odeur | Seuil olfactif (ppb) | Ion spécifique m/z |
|---|---|---|---|---|---|
| 1 | Pentanal | 6,24 | Verte | 18* | 44 |
| 2 | Hexanal | 8,24 | Herbe coupée, pomme verte | 4,5 | 82 |
| 3 | 1 -octène-3-ol | 17,99 | Champignon-solvant (peinture), champignon-encre | 1 / 0,05* | 57 |
| 4 | 2-pentylfurane | 12,10 | florale | 6 | 81 |
| 5 | Octanal | 13,82 | Floral-agrume | 0,7 | 84 |
| 6 | 3,5-octadiène-2-ol ou 3-octène-2-one | 17,03 | Floral-zeste | 0,1** | 111 |
| 7 | 3,5-octadiène-2-one (2 pics) | 19,96+ 21,24 | florale | 0,1** | 95 |
| 8 | Nonanal | 16,68 | Floral-verte, florale | 1 | 57 |
| 9 | 2-Nonénal | 20,37 | Végétal, huile | 0,08 | 83 |
| 10 | (E,E)-2,4-nonadiénal | 24,42 | Huileuse-oxydée | 0,09 | 81 |
| 11 | Acide hexanoïque | 27,51 | Fromage, rance | 3000 | 60 |

| | | | | | |
|---|---|---|---|---|---|
| * seuil olfactif selon H. Jelen, Journal of Chromatographic Science, vol. 44, august 2006 ** valeur estimée Sans autre indication, le seuil olfactif est repris de www.leffingwell.com/odorthre.htm | | | | | |

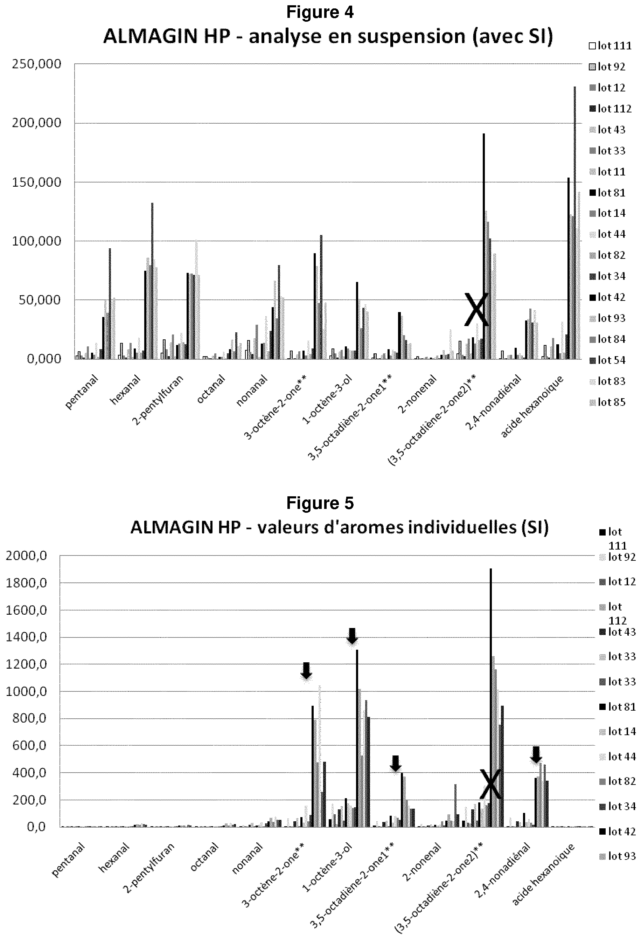

Comme le montre la **Figure 4****,** les produits inacceptables semblent bien plus chargés en ces 11 composés volatils que les échantillons acceptables.

L'analyse statistique confirme que toutes les 11 molécules sont significatives (excepté le deuxième pic du 3,5-octadiène-2-one à 21,24 min).

En conclusion, le suivi de ces 11 molécules (pentanal, hexanal, 1-octène-3-ol, 2-pentylfurane, octanal, 3,5-octadiène-2-ol ou 3-octène-2-one, 3,5-octadiène-2-one (premier des 2 pics), nonanal, 2-nonénal, (E,E)-2,4-nonadiénal, acide hexanoïque) nous permet de distinguer les produits acceptables des produits non-acceptables sur le critère « peinture », par analyse des volatils du produit mis en suspension aqueuse.

### Elaboration du modèle simplifié

Afin de simplifier le modèle, il est décidé de retenir les composés ayant le plus grand impact sur l'odeur globale des compositions de biomasse de microalgues riches en protéines selon l'invention, c'est-à-dire les composés avec des seuils olfactifs extrêmement bas.

Ces valeurs d'arômes individuelles (= concentration du composé/son seuil olfactif) sont représentées dans le **Figure 5****.**

Tenant compte de la concentration et du seuil olfactif de chaque composé, quatre composés semblent particulièrement importants pour les propriétés sensorielles des compositions de biomasse de microalgues riches en protéines selon l'invention : le 3,5-octadiène-2-ol ou 3-octène-2-one (floral-zeste), le 1-octène-3-ol (champignon-solvant, peinture, champignon-encre), le 3,5-octadiène-2-one (le premier des deux pics, florale), et le (E,E)-2,4-nonadiénal (huileuse-oxydée).

Les descripteurs individuels de ces quatre composés rejoignent fort bien l'odeur globale perçue des compositions de biomasse de microalgues riches en protéines selon l'invention inacceptables.

Il est donc possible d'établir une valeur d'arôme globale pour les compositions de biomasse de microalgues riches en protéines, basée sur ces quatre composés :
Valeur globale d'arôme = ∑ des valeurs arômes individuels du 3,5-octadiène-2-ol (ou 3-octène-2-one), du 1-octène-3-ol, du 3,5-octadiène-2-one, et du (E,E)-2,4-nonadiénal.

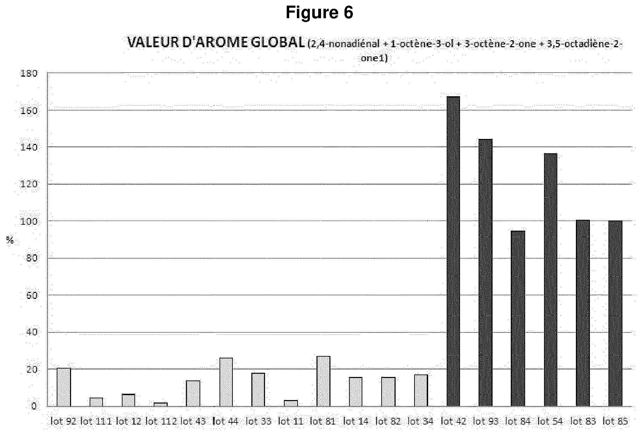

Comme le montre la **Figure 6****,** il est désormais aisé de classer les différents lots de compositions de biomasse de microalgues riches en protéines en deux familles :
- les acceptables : il s'agit des lots 111, 92, 12, 112, 43, 33, 33, 81, 14, 44, 82 et 34 ;
- les inacceptables : il s'agit des lots 83, 84 et 85.

A noter que le lot 85, lot de qualité organoleptique inacceptable selon l'exemple 1, présente une valeur d'arôme de 100 %.

Les lots acceptables présentent donc bien une valeur d'arome globale comprise entre 0 et 40 % par rapport à celle de composition de farine de microalgues de référence organoleptique inacceptable, en l'occurrence ici le lot 85.

Les lots 42, 93 et 54 présentent une valeur globale d'arôme, sur la base des quatre composés organoleptiques, bien supérieure à celle du lot 85 de référence.

Cependant, il faut noter que le lot 85 a été défini inacceptable sur le seul descripteur "peinture".

Les lots 42, 93 et 54, sur le plan de l'analyse des composés organiques volatils, sont particulièrement marqués, sans doute par un effet de synergie entre composés organiques volatils.

Il n'en demeure pas moins que le modèle simplifié basé sur cette sélection des 4 composés organiques volatils sur les 11 de référence permet de classer les compositions de biomasses de microalgues riches en protéines en deux familles distinctes aisément identifiables.

### Description des Figures

**FIGURE 1** : Note moyenne obtenue au descripteur peinture par chacune des compositions.
**FIGURE 2** : Chromatogrammes (TIC) des composés organiques volatils prélevés dans les échantillons par SPME en suspension aqueuse
**FIGURE 3** **:** Intégration de la totalité des pics pour la zone 3,2-35,0 min des chromatogrammes (SPME en suspension aqueuse)
**FIGURE 4** **:** Teneurs relatives en 11 composés sélectionnés prélevés dans l'espace des échantillons par SPME en suspension aqueuse
**FIGURE 5** : Valeurs d'arômes individuelles des 11 composés sélectionnés
**FIGURE 6** : Valeur d'arôme globale basée sur 4 composés

### Aspects

Aspect 1. Méthode pour déterminer la qualité organoleptique d'une composition de biomasse de microalgues riche en protéines comprenant la détermination de la teneur en 11 composés organiques volatils, les 11 composés organiques volatils étant le pentanal, l'hexanal, le 1-octène-3-ol, le 2-pentylfurane, l'octanal, le 3,5-octadiène-2-ol (ou 3-octène-2-one), le 3,5-octadiène-2-one, le nonanal, le 2-nonénal, le (E,E)-2,4-nonadiénal et l'acide hexanoïque, caractérisée en ce que la biomasse de microalgues comprend plus de 50 % de protéines en poids sec de biomasse et en ce que les microalgues sont du genre Chlorella.
Aspect 2. Méthode selon l'aspect 1, dans laquelle la teneur en 11 composés organiques volatils est déterminée par SPME / GC, de préférence par SPME / GC-MS.
Aspect 3. Méthode selon l'aspect 1 ou 2, caractérisée en ce que la teneur en 11 composés organiques volatils est déterminée par la surface des pics de chromatographie après SPME / GC.
Aspect 4. Méthode selon l'un quelconque des aspects 1-3, caractérisée en ce que la teneur en 11 composés organiques volatils, en particulier la surface des pics de chromatographie correspondant aux 11 composés organiques volatils, est comparée à celle de biomasse(s) de microalgues riche en protéines de référence pour laquelle ou lesquelles les qualités organoleptiques sont définies, notamment comme inacceptable ou acceptable.
Aspect 5. Procédé de définition d'un profil analytique en composés organiques volatils permettant d'évaluer la qualité organoleptique des compositions de biomasse de microalgues riches en protéines, comprenant :
   - la construction d'une première matrice associant des compositions de biomasse de microalgues, dont deux témoins de qualité organoleptique acceptable et inacceptable, à l'évaluation de leurs qualités organoleptiques par un panel sensoriel d'au moins 15 personnes,
   - la construction d'une seconde matrice associant à ces mêmes compositions leur caractérisation par un profil d'analyse des composés organiques volatils, et
   - la corrélation de la première matrice à la seconde pour produire un modèle de relation à partir duquel les compositions présentant un profil organoleptique optimisé peuvent être ainsi caractérisées par leur profil analytique en composés organiques volatils ;
   - caractérisée en ce que la biomasse de microalgues comprend plus de 50 % de protéines en poids sec de biomasse et en ce que les microalgues sont du genre Chlorella.
Aspect 6. Procédé selon l'aspect 5, caractérisé en ce que les descripteurs de l'analyse sensorielle comprennent :
   - les odeurs suivantes : végétal, purée, bouillon, beurre rance, fromage, fumier, fermenté, cacahuète et peinture ; et
   - les couleurs suivantes : jaune et vert.
Aspect 7. Méthode pour déterminer la qualité organoleptique d'une composition de biomasse de microalgues riche en protéines comprenant la détermination de la teneur en 4 composés organiques volatils, ces 4 composés organiques étant le 3,5-octadiène-2-ol (ou 3-octène-2-one), le 1-octène-3-ol, du 3,5-octadiène-2-one, et le (E,E)-2,4-nonadiénal et en ce que l'on calcule une valeur d'arôme globale par la somme des valeurs d'arômes individuelles du 3,5-octadiène-2-ol (ou 3-octène-2-one), le 1-octène-3-ol, du 3,5-octadiène-2-one, et le (E,E)-2,4-nonadiénal,
   caractérisée en ce que la biomasse de microalgues comprend plus de 50 % de protéines en poids sec de biomasse et en ce que les microalgues sont du genre Chlorella.
Aspect 8. Méthode de sélection de compositions de biomasse de microalgues riches en protéines présentant un profil organoleptique acceptable, caractérisée en ce que la qualité organoleptique est déterminée par la méthode selon l'un quelconque des aspects 1 à 7 et que la composition est sélectionnée lorsque la valeur d'arôme globale calculée par la méthode selon l'aspect 7 est comprise entre 0 et 40 % par rapport à celle d'une composition de biomasse de microalgues de référence organoleptique inacceptable, caractérisée en ce que la biomasse de microalgues comprend plus de 50 % de protéines en poids sec de biomasse et en ce que les microalgues sont du genre Chlorella.
Aspect 9. Méthode selon l'un quelconque des l'aspects 1-4, et 7, caractérisée en ce que les microalgues sont choisies dans le groupe constitué de *Chlorella vulgaris*, *Chlorella sorokiniana* et *Chlorella protothecoides,* et plus particulièrement *Chlorella protothecoides.*

## Revendications

1. Composition de biomasse de microalgues riche en protéines présentant un profil organoleptique optimisé, dans laquelle la valeur d'arôme globale est faible, de préférence entre 0 et 40 % par rapport à celle d'une composition de biomasse de microalgues de référence organoleptique inacceptable, la valeur d'arôme globale étant la somme des valeurs d'arômes individuelles du 3,5-octadiène-2-ol (ou 3-octène-2-one), le 1-octène-3-ol, du 3,5-octadiène-2-one, et le (E,E)-2,4-nonadiénal.

2. Composition de biomasse de microalgues riche en protéines selon la revendication 1, dans laquelle la biomasse comprend plus de 50 % de protéines en poids sec de biomasse.

3. Composition de biomasse de microalgues riche en protéines selon la revendication 1 ou 2, dans laquelle les microalgues sont du genre Chlorella.

4. Composition de biomasse de microalgues riche en protéines selon l'une quelconque des revendications précédentes, dans laquelle les microalgues sont choisies dans le groupe constitué de *Chlorella vulgaris*, *Chlorella sorokiniana* et *Chlorella protothecoides.*

5. Composition de biomasse de microalgues riche en protéines selon l'une quelconque des revendications précédentes, dans laquelle les microalgues sont *Chlorella protothecoides.*

6. Composition de biomasse de microalgues riche en protéines selon l'une quelconque des revendications précédentes, dans laquelle les microalgues sont du genre Chlorella et sont privées de pigments chlorophylliens.

7. Composition de biomasse de microalgues riche en protéines selon l'une quelconque des revendications précédentes, dans laquelle la valeur d'arômes individuelle est la Concentration du composé divisée par le seuil olfactif du composé.
